(19) Europäisches Patentamt
European Patent Office
Office européen des brevets

(11) Numéro de publication: **0 579 277 A1**

# (12) DEMANDE DE BREVET EUROPEEN

(21) Numéro de dépôt: **93114975.1**

(51) Int. Cl.[5]: **C07D 233/54**, A61K 31/415

(22) Date de dépôt: **25.03.93**

This application was filed on 17 - 09 - 1993 as a divisional application to the application mentioned under INID code 60.

(30) Priorité: **30.03.92 FR 9203828**

(43) Date de publication de la demande: **19.01.94 Bulletin 94/03**

(60) Numéro de publication de la demande initiale en application de l'article 76 CBE : **0 565 396**

(84) Etats contractants désignés: **AT BE CH DE DK ES FR GB GR IE IT LI LU MC NL PT SE**

(71) Demandeur: **SYNTHELABO**
**22, Avenue Galilée**
**F-92350 Le Plessis Robinson(FR)**

(72) Inventeur: **Lassalle, Gilbert**
**53 avenue Victor Hugo**
**F-92140 Clamart(FR)**
Inventeur: **Purcell, Thomas**
**47 bis rue de la Millière Les Mesnuls**
**F-78490 Montfort l'Amaury(FR)**
Inventeur: **Galtier, Daniel**
**16 rue Francis Poulenc**
**F-78280 Guyancourt(FR)**
Inventeur: **Williams, Paul Howard**
**24 rue Saint Bernard**
**F-75011 Paris(FR)**
Inventeur: **Galli, Frédéric**
**3 Résidence de l'Orangerie**
**F-78170 la Celle St Cloud(FR)**

(74) Mandataire: **Thouret-Lemaitre, Elisabeth et al**
**SYNTHELABO,**
**Service Brevets,**
**B.P. 72**
**F-92352 Le Plessis-Robinson Cédex (FR)**

## (54) Dérivés d'imidazole, leur préparation et leur utilisation comme intermédiaires de synthèse.

(57) Composés de formule (I)

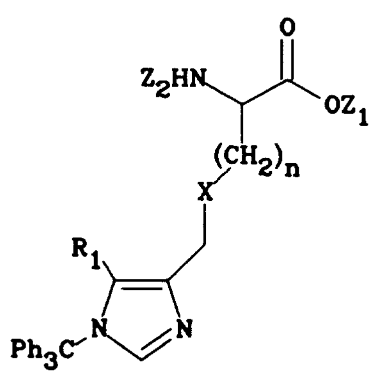

(I)

dans laquelle
$R_1$ représente soit un atome d'hydrogène, soit un groupe ($C_1$-$C_4$)alkyle,
X représente soit un atome de soufre, soit un atome d'oxygène, soit un groupe méthylène,
$Z_1$ représente soit un atome d'hydrogène, soit un groupe 1,1-diméthyléthyle,
$Z_2$ représente soit un atome d'hydrogène, soit un groupe triphénylméthyle, soit un groupe phénylméthoxycarbo-

EP 0 579 277 A1

nyle et n = 1 ou 2,
ainsi que leurs sels d'addition aux acides.
Intermédiaires de synthèse.

La présente invention a pour objet des dérivés d'imidazole, leur préparation et leur utilisation comme intermédiaires de synthèse.

Les composés de l'invention répondent à la formule (I)

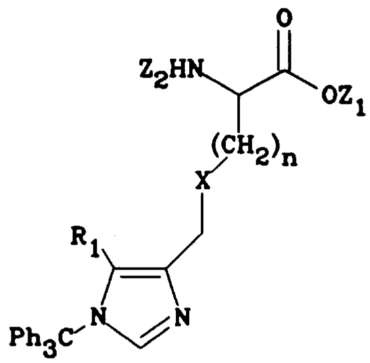

(I)

dans laquelle

$R_1$ représente soit un atome d'hydrogène, soit un groupe $(C_1\text{-}C_4)$alkyle,

X représente soit un atome de soufre, soit un atome d'oxygène, soit un groupe méthylène,

$Z_1$ représente soit un atome d'hydrogène, soit un groupe 1,1-diméthyléthyle,

$Z_2$ représente soit un atome d'hydrogène, soit un groupe triphénylméthyle, soit un groupe phénylméthoxycarbonyle et n = 1 ou 2.

Les composés de l'invention possèdent un centre asymétrique au niveau de la partie aminée centrale dont la configuration préférentielle est [S].

Les composés selon l'invention peuvent exister sous forme de bases libres ou de sels d'addition aux acides.

Dans les schémas 1 à 4 qui suivent Ts représente un groupe 4-(méthylphényl)sulfonyle.

Les composés de l'invention pour lesquels X représente un groupe méthylène répondent à la formule (Ia)

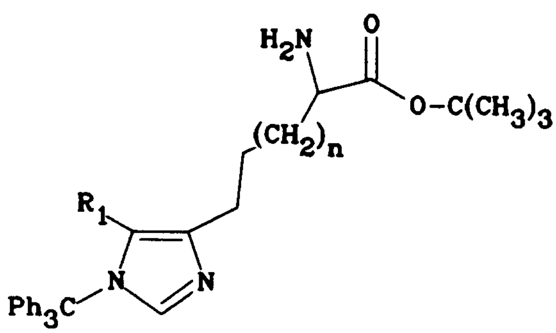

(Ia)

dans laquelle $R_1$ et n sont tels que définis précédemment; ils peuvent être synthétisés selon le schéma 1 de la page suivante.

Dans une première étape, on fait réagir un alcool de formule (II) avec du chlorure de thionyle dans un mélange de diméthylformamide/dichlorométhane puis on fait réagir le composé obtenu avec de la triphénylphosphine dans un solvant tel que le diméthylformamide ou le benzène à une température de 80 °C; on obtient un chlorure de triphénylméthyl phosphonium de formule (III).

Dans une deuxième étape, on fait réagir le composé de formule (III) avec un composé de formule (IV), dans un solvant tel que le tétrahydrofurane, en présence de n-butyllithium à une température de - 70 °C. On obtient un composé de formule (V), sous forme d'un mélange cis/trans au niveau de la double liaison.

Dans une troisième étape, on soumet le composé (V) obtenu précédemment à une hydrogénation catalytique pour obtenir un composé de formule (Ia) dont on peut déprotéger la partie C-terminale en le traitant par de l'acide chlorhydrique gazeux dans un solvant tel que le benzène.

**Schéma 1**

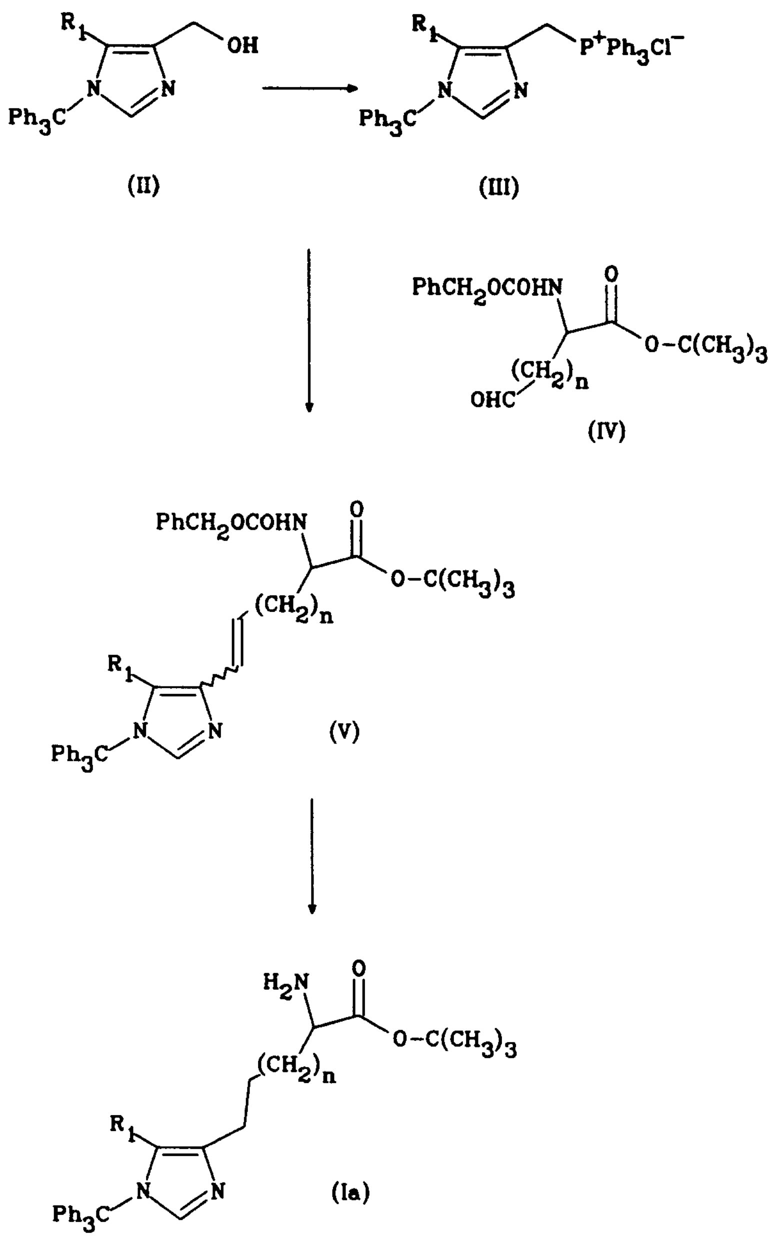

(II) (III) (IV) (V) (Ia)

Les composés de l'invention pour lesquels X représente un atome de soufre et n = 1 répondent à la formule (Ib)

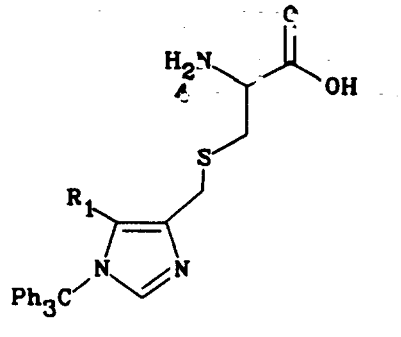

**(Ib)**

dans laquelle $R_1$ est tel que défini précédemment. Les composés répondant à la formule (Ib) peuvent être synthétisés selon le schéma 2.

On fait réagir un alcool de formule (II) avec du chlorure de thionyle dans un mélange diméthylformamide/dichlorométhane puis on fait réagir le composé obtenu avec de la *L*-cystéine de formule (XI) dans une solution aqueuse 1 N d'hydroxyde de sodium.

## Schéma 2

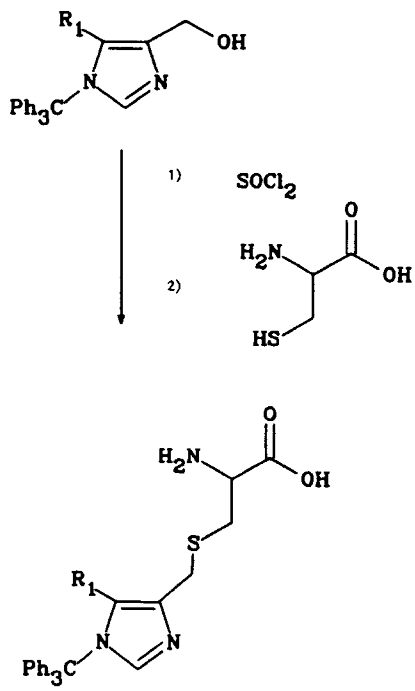


(II)

(XI)

(Ib)

Les composés de l'invention pour lesquels X représente un atome de soufre et n = 2 répondent à la formule (Ic)

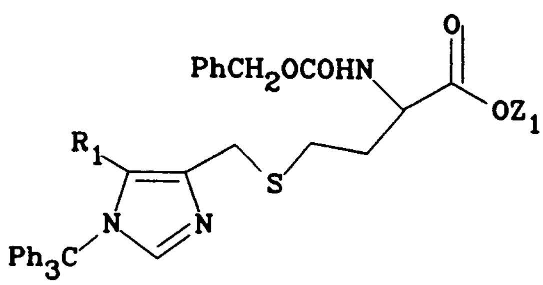

(Ic)

dans laquelle $R_1$ et $Z_1$ sont tels que définis précédemment. Les composés répondant à la formule (Ic) peuvent être

**Schéma 3**

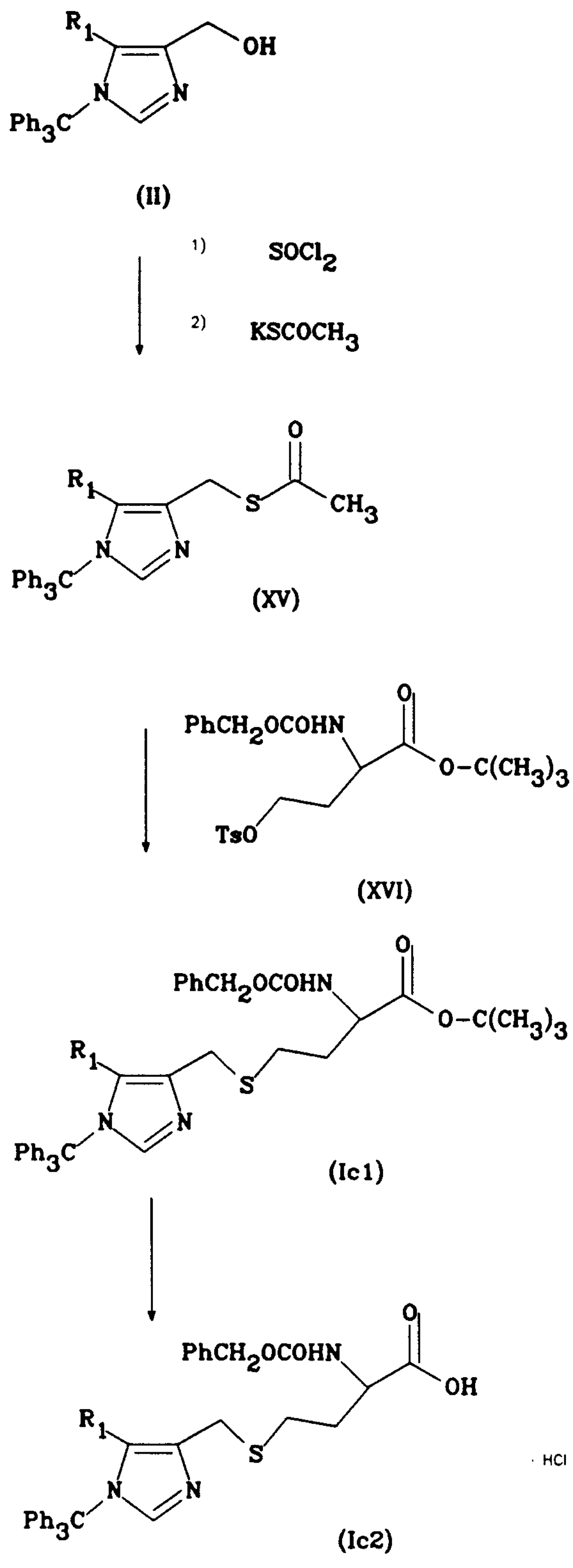


(II)

(XV)

(XVI)

(Ic1)

(Ic2)

synthétisés selon le schéma 3 de la page précédente. Dans une première étape, on fait réagir un alcool de formule (II) avec du chlorure de thionyle dans un mélange diméthylformamide/dichlorométhane puis on fait réagir le composé obtenu avec du thioacétate de potassium. On obtient un composé de formule (XV).

Dans une deuxième étape, on fait réagir le composé de formule (XV) avec un tosylate de formule (XVI) pour obtenir un composé de formule (Ic1); cette réaction est conduite en présence d'une base comme le

méthylate de sodium. Le tosylate de formule (XVI) est obtenu par action du chlorure de paratoluène-sulfonyle, en présence d'une base, sur l'alcool précurseur du composé de formule (IV) dont la synthèse est décrite par Valerio R.M. et coll. dans Synthesis, 1988, 786. Dans une troisième étape, on déprotège la partie C-terminale du composé de formule (Ic1) en le traitant par de l'acide chlorhydrique gazeux dans un solvant tel que le benzène et on obtient un composé de formule (Ic2) dont on peut déprotéger la partie N-terminale par hydrogénation catalytique.

Les composés de l'invention pour lesquels X représente un atome d'oxygène répondent à la formule (Id)

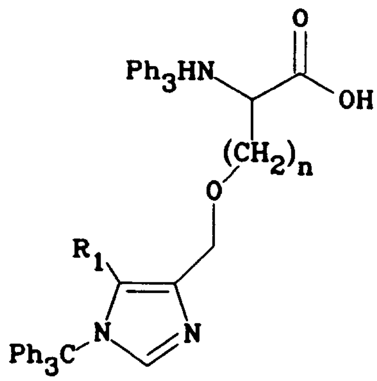

(Id)

dans laquelle $R_1$ et n sont tels que définis précédemment; les composés de formule (Id) peuvent être synthètisés selon le schéma 4 de la page suivante.

On fait réagir un alcool de formule (II) avec du chlorure de thionyle dans un mélange de diméthylformamide et de dichlorométhane puis on fait réagir le composé obtenu avec un composé de formule (XXII) préalablement traité en présence d'un excès d'hydrure de sodium dans le tétrahydrofurane et on obtient le

**Schéma 4**

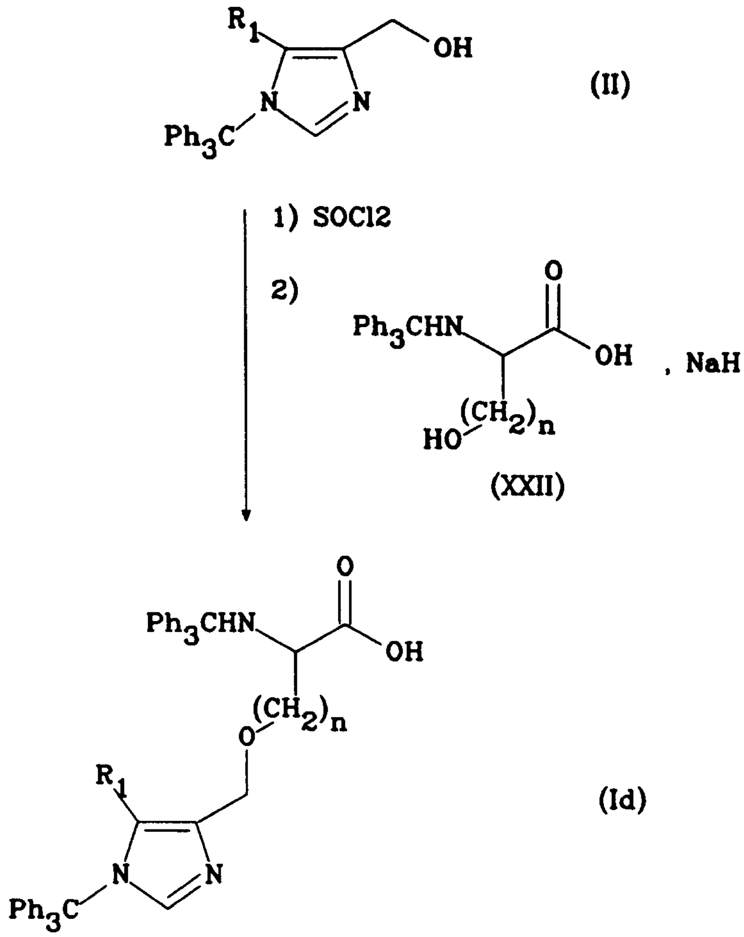


(II)

(XXII)

(Id)

composé de formule (Id) dont on peut déprotéger la partie N-terminale par action de l'acide trifluoroacétique dans un mélange dichlorométhane/méthanol.

Le brevet européen 0008746 décrit des dérivés de $N^2$-arylsulfonyl-*L*-argininamide.

Les composés de départ sont disponibles dans le commerce ou décrits dans la littérature ou peuvent être préparés selon des méthodes qui y sont décrites ou qui sont connues de l'homme du métier.

Ainsi les composés de formule (II) sont préparés par des méthodes analogues à celles décrites dans le brevet européen 0242973 et à celles décrites par Griffith R.K. et coll., Synthesis, 1983, 576.

Certains composés de formule (IV) sont décrits par Valerio R.N. et coll. dons Synthesis, 1988, 786. Les composés de formule (XXII) sont décrits par Barlos et coll dans J. Org. Chem., 1982, 47, 1324 et traités comme il est décrit par Barlos et coll. dans Tetrahedron, 1983, 39, 475.

Les exemples suivants illustrent la préparation de certains composés conformément à l'invention. Les microanalyses élémentaires confirment la structure des composés obtenus.

Exemple 1

chlorhydrate de (*S*)-2-amino-5-[1-(triphénylméthyl)-1*H*-imidazol-4-yl]pentanoate de 1,1-diméthyléthyle

1.1. chlorure de triphényl[[(1-triphénylméthyl)-1*H*-imidazol-4-yl]méthyl]phosphonium

A 670 ml d'une solution de 105,5 g (294 mmoles) de 4-(chlorométhyl)-1-(triphénylméthyl)-1*H*-imidazole dans le diméthylformamide, on ajoute 77,7 g (296 mmoles de triphénylphosphine. On chauffe à 80 °C pendant 3 heures. On évapore le solvant, on reprend le brut dans l'éther et on le triture. On filtre le précipité et on le sèche sous vide sur du pentoxyde de phosphore.

On obtient 162 g de produit sous forme de cristaux jaunâtres.

| | |
|---|---|
| Point de fusion = 210 °C | Rendement = 89 % |

1.2. (*S*,*E*)-2-[[(phénylméthoxy)carbonyl]amino]-5-(1-(triphénylméthyl)-1*H*-imidazo1-4-yl]pent-4-énoate de 1,1-diméthyléthyle

Dans un ballon tricol, sous argon, on introduit 50,93 g (820 mmoles) de chlorure de triphényl[[1-triphénylméthyl)-1*H*-imidazol-4-yl]méthyl]phosphonium en solution dans 333 ml de tétrahydrofurane. On ajoute goutte à goutte, à - 70 °C, 51,2 ml d'une solution 1,6 M de n-butyllithium dans l'hexane (820 mmoles). Aprés 30 minutes d'agitation à - 70 °C, on verse rapidement le milieu réactionnel dans 270 ml d'une solution 0,253 M, refroidie à 70 °C, de (*S*)-4-oxo-2-[[(phénylméthoxy)-carbonyl]amino]butanoate de 1,1-diméthyléthyle dans le tétrahydrofurane (683 mmoles). On laisse remonter le mélange à la température ambiante pendant une nuit. On hydrolyse le mélange avec 280 ml d'une solution aqueuse saturée de chlorure de sodium. On sépare la phase aqueuse de la phase organique et on l'extrait par 2 fois 140 ml d'acétate d'éthyle. On réunit les phases organiques, on les sèche sur du sulfate de magnésium et on évapore à sec. On purifie par chromatographie sur colonne de gel de silice en éluant par un gradient hexane/acétate d'éthyle.
On obtient un mélange d'oléfine cis et trans.
Pour la forme cis:
Point de fusion = 66 °C
$R_f$ = 0,30 [hexane/acétate d'éthyle (60/40)] Pour la forme trans:
$R_f$ = 0,15 [hexane/acétate d'éthyle (60/40)] Rendement = 40 %

1.3. chlorhydrate de (*S*)-2-amino-5-[1-(triphénylméthyl)-1*H*-imidazol-4-yl]pentanoate de 1,1-diméthyléthyle

On met en solution 5,83 g (9,50 mmoles) du composé cis obtenu précédemment en 1.2, dans 120 ml d'éthanol. On réalise, pendant 5 heures, une hydrogénation catalytique, à 50 psi, en présence de palladium sur charbon comme catalyseur. On filtre le catalyseur sur un mélange célite/silice et on évapore le solvant. On recueille 4,32 g de produit que l'on dissout dans 90 ml d'alcool isopropylique "chlorhydrique" (HCl = 0,1 N) chaud. On évapore le solvant, on précipite par de l'éther et on sèche le produit sous vide.
On obtient 3,62 g de produit.

| | |
|---|---|
| Point de fusion = 73 °C | Rendement = 73 % |

Exemple 2

acide 2-amino-3-[[[1-(triphénylméthyl)-1*H*-imidazol-4-yl]méthyl]thio]propanoïque

A 40 ml d'une solution aqueuse 1 N d'hydroxyde de sodium (40 mmoles), on ajoute 2,66 g (22 mmoles) de *L*-cystéine. On agite et on ajoute, sous agitation, à 0 °C, une solution de 7,2 g de 4-(chlorométhyl)-1-(triphénylméthyl)-1*H*-imidazole (20 mmoles) dans 50 ml d'éthanol et 20 ml de tétrahydrofurane. On laisse remonter à la température ambiante et on agite pendant 1 heure. On évapore l'éthanol et le tétrahydrofurane sous pression réduite. On reprend le résidu dans 100 ml d'eau et 20 ml d'acide chlorhydrique 1 N. On filtre le précipité obtenu, on le lave à l'eau et on le sèche. On obtient 8 g de produit.
Point de fusion = 162-164 °C (décomposition)
Rendement = 90 %

Exemple 3

chlorhydrate de l'acide (*S*)-2-[[(phénylméthoxy)carbonyl]amino]-4-[[[1-(triphénylméthyl)-1*H*-imidazol-4-yl]-méthyl]thio]butanoïque

3.1. (*S*)-2-[[(phénylméthoxy)carbonyl]amino]-4-[[[1-(triphénylméthyl)-1*H*-imidazol-4-yl]méthyl]thio]butanoate de 1,1-diméthyléthyle

A une solution de 3,58 g (10 mmoles) de 4-(chlorométhyl)-1-(triphénylméthyl)-1*H*-imidazole dans 40 ml d'éthanol, sous azote, on ajoute 6 g de thioacétate de potassium. On agite par sonication pendant 15 minutes, puis on verse le milieu réactionnel sur de l'éther. On lave la phase organique à l'eau, puis par une solution saturée d'hydrogénocarbonate de sodium puis par une solution saturée de chlorure de sodium. On sèche sur du sulfate de magnésium et on évapore le solvant sous pression réduite. On reprend le résidu dans 100 ml de méthanol dégazé à l'azote et contenant 1,9 ml d'une solution 5,3 N de méthylate de sodium (10 mmoles). On agite pendant 15 minutes et on ajoute 4,63 g (10 mmoles) de (*S*)-4-[[(4-méthylphényl)sulfonyl]oxy]-2-[[(phénylméthoxy)carbonyl]-amino]butanecarboxylate de 1,1-diméthyléthyle dans 40 ml de méthanol dégazé. On agite pendant 24 heures à la température ambiante. On essore le précipité obtenu, on le lave au méthanol et à l'eau. On le sèche sur du sulfate de magnésium. On obtient 3 g de produit.

| | |
|---|---|
| Point de fusion = 183-185 °C | Rendement = 50 % |

3.2. chlorhydrate de l'acide (*S*)-2-[[(phénylméthoxy)carbonyl]amino]-4-[[[1-(triphénylméthyl)-1*H*-imidazol-4-yl)-méthyl]thio]butanoïque

On place 2,5 g (3,86 moles) du composé obtenu précédemment en 3.1 dans 50 ml de benzène. On sature la solution en acide chlorhydrique gazeux, à l'abri de l'humidité, à 0 °C, pendant 10 minutes. On agite le milieu réactionnel pendant 2 heures à 0 °C. On évapore sous pression réduite.
On obtient 2,2 g de produit que l'on utilisera tel quel pour la prochaine étape.

| | |
|---|---|
| Point de fusion = 78-82 °C | Rendement = 91 % |

Exemple 4

(*N*)-(triphénylméthyl)-(*O*)-[[1-(triphényl-méthyl)-1*H*-imidazol-4-yl]méthyl]-(*L*)-sérine

A une suspension de 5,8 g (144,2 mmoles) d'hydrure de sodium à 60 % dans 112 ml de tétrahydrofurane, on ajoute, à - 15 °C, 0,39 g (5,7 mmoles) de 4-(chlorométhyl)-1-(triphénylméthyl)-1*H*-imidazole puis 10,01 g (28,83 mmoles) de (*N*)-(triphénylméthyl)-(*L*)-sérine. On laisse à -15 °C pendant 45 minutes et on ajoute 12 g (34,6 mmoles) de 4-(chlorométhyl)-1-(triphénylméthyl)-1*H*-imidazole. On agite pendant 2 heures à 0 °C puis pendant 5 heures à la température ambiante. On refroidit le milieu réactionnel à 0 °C, on dilue par 200 ml d'eau et on neutralise par addition de 6,59 ml (115 mmoles) d'acide acétique. On laisse décanter, on recueille la phase aqueuse et on l'extrait par 2 fois 200 ml d'acétate d'éthyle. On réunit les phases organiques, on les lave par environ 300 ml d'eau et on les sèche sur du sulfate de magnésium. On évapore les solvants et on purifie le résidu par chromatographie sur colonne de gel de silice en éluant par un gradient éthanol/dichlorométhane (2/98 à 4/96).
On obtient 12,82 g de produit.

| | |
|---|---|
| Point de fusion = 104-106 °C | Rendement = 68 % |

Les composés selon l'invention sont utiles comme substituts de lysine et d'arginine utilisables dans la synthèse de peptides et de pseudopeptides conformément à l'exemple A cidessous.

Exemple A

[2*R*-[1(*S*),2α,4β]]-4-méthyl-1-[2-[[(3-méthyl-1,2,3,4-tétrahydroquinoléin-8-yl)sulfonyl]amino]-1-oxo-5-(1*H*-imidazol-4-yl)pentyl]pipéridine-2-carboxylate d'éthyle

1. (*S*)-2-[[(3-méthylquinoléin-8-y1)sulfonyl]amino]-5-[1-(triphénylméthyl)-1*H*-imidazol-4-yl]pentanoate de 1,1-diméthyléthyle

A 3,8 g (7,26 mmoles) du composé obtenu précédemment dans l'exemple 1.3, on ajoute 1,76 g (7,28 mmoles) de 8-(chlorosulfonyl)-3-méthylquinoléine en solution dans 50 ml de chloroforme, en présence de 2,1 ml (14,5 mmoles) de triéthylamine, à 5 °C. On laisse sous agitation pendant 3 heures, on sépare la phase organique, on la lave par une solution d'acide chlorhydrique 0,1 N et on évapore. On purifie le brut par chromatographie sur colonne de gel de silice en éluant par un mélange éthanol/dichlorométhane (5/95). On obtient 3,6 g de produit.

| | |
|---|---|
| Point de fusion = 56 °C | Rendement = 72 % |

2. acide (*S*)-2-[[(3-méthylquinoléin-8-yl)sulfonyl]amino]-5-[1-(triphénylméthyl)-1*H*-imidazol-4-yl]pentanoïque

Dans une solution de 2,33 g (3,39 mmoles) du composé obtenu dans l'étapt précédente dans 34 ml de benzène, refroidie à 0 °C, sous azote, on fait barboter, pendant 15 minutes, de l'acide chlorhydrique gazeux. On laisse le milieu réactionnel revenir à la température ambiante et on maintient l'agitation pendant 2 heures à cette température. On évapore le solvant sous vide et on purifie le résidu par chromatographie sur colonne de gel de silice en éluant par un mélange éthanol/dichlorométhane (20/80).
On obtient 1,42 g de produit sous forme d'une poudre blanchâtre.

| | |
|---|---|
| Point de fusion = 170 °C | Rendement = 66 % |

3. [2*R*-[1(*S*),2α,4β]]-4-méthy1-1-[2-[[(3-méthylquinoléin-8-yl)sulfonyl]amino]-1-oxo-5-[1-(triphénylméthyl)-1*H*-imidazol-4-yl]pentyl]pipéridine-2-carboxylate d'éthyle

A une suspension de 2,23 g (3,53 mmoles) du composé obtenu dans l'étape précédente dans 50 ml d'acétonitrile, on ajoute, à 0 °C, sous azote, 1,56 g (3,53 mmoles) d'hexafluorophosphate de [(benzotriazol-1-yl)oxy]tris(diméthylamino)phosphonium, 0,78 ml (7,06 mmoles) de 4-méthylmorpholine, 0,61 g (3,57 mmoles) de (2*R*−*trans*)-4-méthylpipéridine-2-carboxylate d'éthyle, préparée comme il est décrit dans le brevet européen 0008746, et 30 ml de dichlorométhane. On laisse, sous agitation, à la température ambiante, pendant 5 heures. On hydrolyse le milieu réactionnel par une solution saturée de chlorure de sodium et on l'extrait avec du chloroforme. On lave la phase organique par une solution d'acide chlorhydrique 0,1 N, puis par une solution saturée d'hydrogénocarbonate de sodium, puis par de l'eau et finalement par une solution de chlorure de sodium. On sèche sur du sulfate de magnésium. On purifie le brut obtenu par chromatographie sur colonne de gel de silice en éluant par un mélange éthanol/dichlorométhane (5/95).
On obtient 1,94 g de produit.
$R_f$ = 0,68 (dichlorométhane/éthanol 95/5)
Rendement = 70 %

4. [2*R*-[1(*S*),2α,4β]]-4-méthyl-1-[2-[[(3-méthyl-1,2,3,4-tétrahydroquinoléin-8-yl)sulfonyl]amino)-1-oxo-5-(1*H*-imidazol-4-yl)pentyl]pipéridine-2-carboxylate d'éthyle

A 1,94 g (2,47 mmoles) de l'ester obtenu dans l'étape précédente, on ajoute 70 ml d'éthanol et 17 ml d'acide acétique. On réalise une hydrogénation catalytique, en présence de palladium sur charbon, à 80 °C, pendant 6 heures. On filtre le mélange et on évapore le solvant. On reprend le résidu obtenu par de l'acide chlorhydrique 1 N, on le lave par de l'éther et on l'extrait par de acétate d'éthyle. On obtient 1,05 g de produit.

Point de fusion = 104 °C (chlorhydrate)
Rendement = 78 %
$[\alpha]_D^{20}$ = + 101 (c = 0,2; méthanol)
Ces peptides et pseudopeptides peuvent être utiles dans toutes les indications cliniques liées à la thrombose ou dans celles où des complications thrombotiques pourraient intervenir, ainsi qu'il est décrit dans la demande de brevet européen 93400772.5.

**Revendications**

**1.** Composés de formule (I)

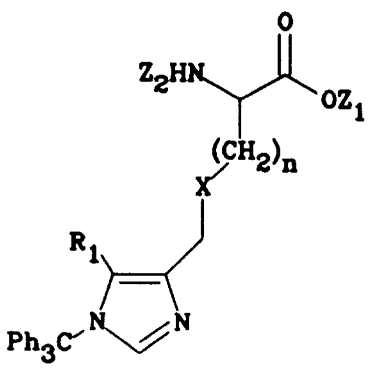

(I)

dans laquelle
$R_1$ représente soit un atome d'hydrogène, soit un groupe ($C_1$-$C_4$) alkyle,
X représente soit un atome de soufre, soit un atome d'oxygène, soit un groupe méthylène,
$Z_1$ représente soit un atome d'hydrogène, soit un groupe 1,1-diméthyléthyle,
$Z_2$ représente soit un atome d'hydrogène, soit un groupe triphénylméthyle, soit un groupe phénylméthoxycarbonyle et n = 1 ou 2,
ainsi que leur sels d'addition aux acides.

**2.** Composés selon la revendication 1 caractérisés en ce que la configuration préférentielle de la partie aminée centrale est [S].

Office européen des brevets

# RAPPORT DE RECHERCHE EUROPEENNE

Numero de la demande

EP 93 11 4975

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int. Cl.5 ) |
| A | EP-A-0 008 596 (DEVINTER EUROPE)<br>* page 3, ligne 11 - ligne 16; exemple 2 *<br>--- | 1 | C07D233/54<br>A61K31/415 |
| A | EP-A-0 460 679 (BANYU PHARMACEUTICAL)<br>* page 109, ligne 22 - ligne 30 *<br>----- | 1 | |
| | | | DOMAINES TECHNIQUES RECHERCHES (Int. Cl.5 ) |
| | | | C07D<br>A61K |

Le présent rapport a été établi pour toutes les revendications

| Lieu de la recherche | Date d'achèvement de la recherche | Examinateur |
|---|---|---|
| LA HAYE | 18 OCTOBRE 1993 | VOYIAZOGLOU D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)